# EUROPEAN PATENT SPECIFICATION

(11) **EP 3 302 540 B1**
(45) Date of publication and mention of the grant of the patent: **07.01.2026**
(21) Application number: 16800841.5
(22) Date of filing: 27.05.2016
(51) Int. Cl.: A61K 39/00, A61K 39/395, C12N 15/861

(54) **ADENO-ASSOCIATED VIRUS MEDIATED DELIVERY OF C1EI AS A THERAPY FOR ANGIOEDEMA**
DURCH ADENO-ASSOZIIERTE VIREN VERMITTELTE FREISETZUNG VON C1EI ALS THERAPIE FÜR ANGIOÖDEM
ADMINISTRATION À MÉDIATION PAR UN VIRUS ADÉNO-ASSOCIÉ DE C1EI EN TANT QUE TRAITEMENT CONTRE L'ANGIO- DÈME

(30) Priority: 28.05.2015 US 201562167603 P; 18.04.2016 US 201662324183 P
(43) Date of publication of application: 11.04.2018
(73) Proprietor: Cornell University, Ithaca, New York 14850 (US)
(72) Inventor: CRYSTAL, Ronald, G., New York, NY 10021 (US); PAGOVICH, Odelya, E., New York, NY 10021 (US); CHIUCHIOLO, Maria, J., Washington, DC 20008 (US)
(74) Representative: Maiwald GmbH
(86) International application number: PCT/US2016/034852
(87) International publication number: WO 2016/191746

(56) References cited:
- EP-A1- 1 598 428
- WO-A1-2014/113701
- WO-A1-2015/035190
- WO-A2-2012/122025
- US-A1- 2003 073 652
- US-A1- 2003 140 358
- US-A1- 2014 228 300
- FRANCISCO A BRACHO: "Hereditary angioedema :", CURRENT OPINION IN HEMATOLOGY, vol. 12, no. 6, 1 November 2005 (2005-11-01), US, pages 493 - 498, XP055462045, ISSN: 1065-6251, DOI: 10.1097/01.moo.0000179805.57486.4e
- DE LA CRUZ ET AL.: "Analysis of SERPING1 expression on hereditary angioedema patients: Quantitative analysis of full-length and exon3 splicing variants", IMMUNOLOGY LETTERS, vol. 141, no. 2, 4 October 2011 (2011-10-04), pages 158 - 164, XP055332266

## Description

### CROSS-REFERENCE TO RELATED APPLICATIONS

This patent application claims the benefit of U.S. Provisional Patent Application No. 62/167,603 filed on May 28, 2015 and U.S. Provisional Patent Application No. 62/324,183 filed April 18, 2016 and U.S. Patent Application No. 15/167,729 filed May 27, 2016.

### INCORPORATION-BY-REFERENCE OF MATERIAL SUBMITTED ELECTRONICALLY

Incorporated by reference in its entirety herein is a computer-readable nucleotide/amino acid sequence listing submitted concurrently herewith and identified as follows: One 41,866 Byte ASCII (Text) file named "724068_ST25.TXT," created on May 27, 2016.

### BACKGROUND OF THE INVENTION

Hereditary angioedema (HAE) is a rare and potentially life-threatening genetic condition characterized by recurrent episodes of swelling that most often affect the skin or mucosal tissues of the upper respiratory and gastrointestinal tracts (see e.g., Banerji, Ann Allergy Asthma Immunol, 111: 329-336 (2013) and Aygoren-Pursun et al., Orphanet J Rare Dis.,9: 99 (2014)). The disease is inherited in an autosomal dominant pattern and affects 1:10,000 to 1:50,000 people. The underlying cause of HAE (type I and II) is attributed to autosomal dominant inheritance of mutations in the C1 esterase inhibitor gene (C1EI gene or SERPING1 gene), mapped to chromosome 11. Eighty-five percent of HAE cases are type I in which there is a deficiency in the amount of C1 esterase inhibitor produced (see e.g., Gower et al., World Allergy Organ J., 4: S9-S21 (2011); Cungo et al., Trends Mol Med, 15: 69-78 (2009); Gooptu et al., Annu Rev Biochem, 78: 147-176 (2009); and Zuraw et al., J Allergy Clin Immunol Pract, 1: 458-467 (2013)). The remainder of cases are characterized by the expression of a dysfunctional C1 esterase inhibitor.

The frequency, duration and severity of attacks associated with HAE vary, with 30% of patients reporting a frequency of greater than one attack/month, 40% report 6 to 11 attacks/year and the remaining 30% are infrequently symptomatic. Usually, symptoms are transient progressing over 12 to 36 hours and subsiding within 2 to 5 days; however, some attacks may last up to one week. Although HAE episodes are self-limiting, the unpredictable occurrence of attacks places considerable strain on patients, often heavily impacting quality of life, and can be fatal.

To date, therapeutic agents are indicated for long-term prophylaxis, therapy for acute attacks and short-term prophylaxis (i.e., prior to dental surgery), and include agents such as Danazol, which has a high adverse effect profile, C1 inhibitor replacement protein, bradykinin receptor antagonists, kallikrein inhibitors, fresh frozen plasma and purified C1 inhibitor. These therapies can alleviate symptoms and maximize quality of life; however, disease recurrence and the need for long-term continued administration remains a major obstacle to therapy (see e.g., Aberer, Ann Med, 44: 523-529 (2012); Charignon et al., Expert Opin Pharmacother, 13: 2233-2247 (2012); Papadopoulou-Alataki, Curr Opin Allergy Clin Immunol, 10: 20-25 (2010); Parikh et al., Curr Allergy Asthma Rep, 11: 300-308 (2011); Tourangeau et al., Curr Allergy Asthma Rep, 11: 345-351 (2011); Bowen et al., Ann Allergy Asthma Immunol, 100: S30-S40 (2008); Frank, Immunol Allergy Clin North Am, 26: 653-668 (2006); Cicardi et al., J Allergy Clin Immunol, 99: 194-196 (1997); Kreuz et al., Transfusion 49: 1987-1995 (2009); Bork et al., Ann Allergy Asthma Immunol, 100: 153-161 (2008); and Cicardi et al., J Allergy Clin Immunol, 87: 768-773 (1991)).

EP 1 598 428 A1 relates to gene therapy methods for treating hereditary angioedema type III. US 2003/0140358 A1 relates to transgenic nonhuman mammals expressing C1 inhibitor in their milk. WO 2012/122025 relates to methods of generating conditionally expressing one or more proteins under the control of a gene expression modulation.

Thus, there is a need for a novel long-lasting therapeutic approach to treat angioedema associated with C1 esterase inhibitor deficiency. This invention provides such a therapeutic approach to treat angioedema.

### BRIEF SUMMARY OF THE INVENTION

The invention provides a chimeric or pseudotyped vector comprising a promoter operably linked to a nucleic acid sequence which encodes human C1 esterase inhibitor (C1EI), optionally in a composition, for use in treating or preventing hereditary angiodema in a mammal. Further embodiments are apparent from the appended claims.

### BRIEF DESCRIPTION OF THE SEVERAL VIEWS OF THE DRAWING(S)

The patent or application file contains at least one drawing executed in color. Copies of this patent or patent application publication with color drawing(s) will be provided by the Office upon request and payment of the necessary fee.

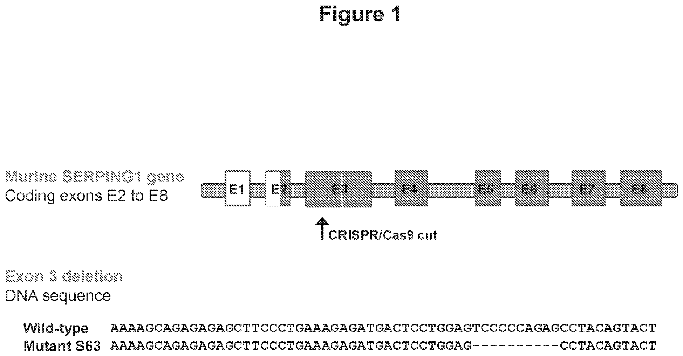

Figure 1 is a schematic of the method used to generate the S63 mouse model of C1EI deficiency generated with SERPING1 E3 targeted mutations.

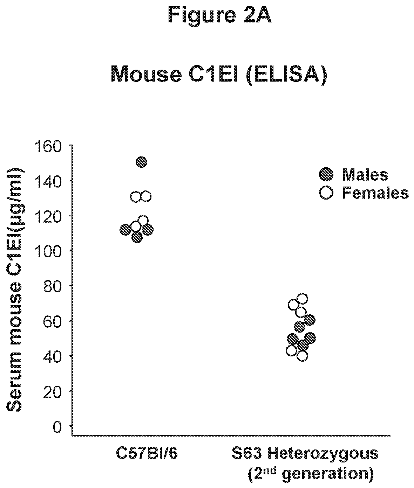

Figure 2A is a graph illustrating the level of C1EI in wild-type and S63 SERPING1^{+/-} heterozygous mice. Mutant S63 and wild-type C1EI levels were measured by ELISA.

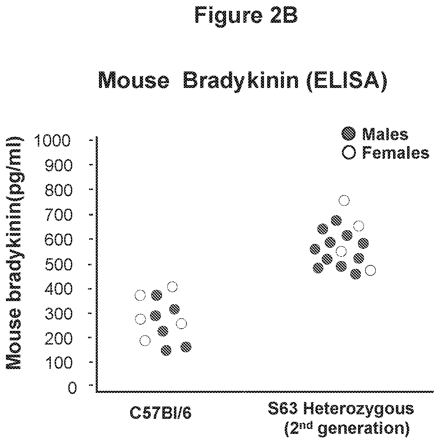

Figure 2B is a graph illustrating bradykinin levels in wild-type and S63 SERPING1^{+/-} heterozygous mice. Mutant S63 and wild-type bradykinin levels were measured by ELISA.

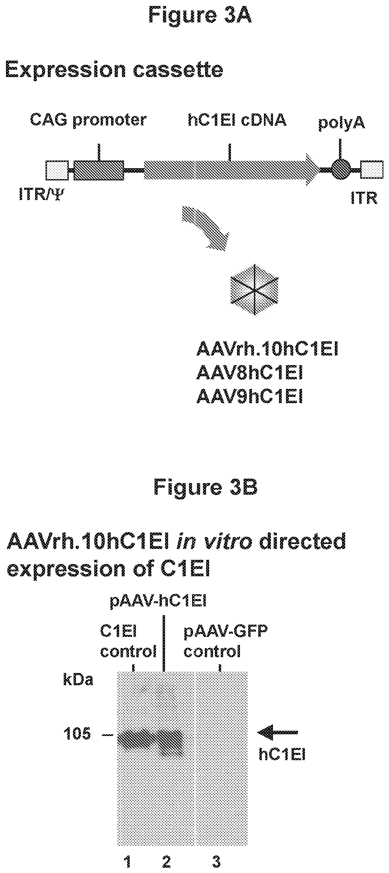

Figure 3A is a schematic of the AAVrh.10hC1EI, AAV8hC1EI, or AAV9hC1EI vector, which depicts the AAV2 inverted terminal repeats (ITR), encapsidation signal (ψ), CMV enhancer/chicken beta-actin (CAG) promoter, optimized human C1EI (hC1EI) cDNA, and rabbit β-globulin polyadenylation signal.
Figure 3B is an image of a Western blot which depicts expression of hC1E1 encoded by the AAV-hC1EI plasmid in HEK 293T cells.

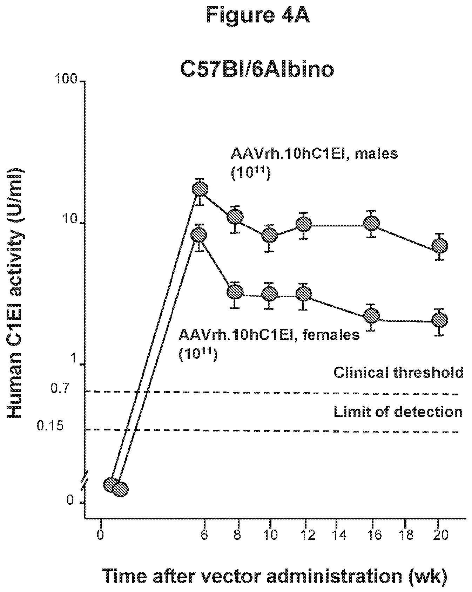

Figure 4A is a graph of experimental data illustrating the long term expression of human C1EI following a single intravenous administration of the AAVrh. 10hC1EI vector to wild-C57Bl/6Albino mice (n=5/group).

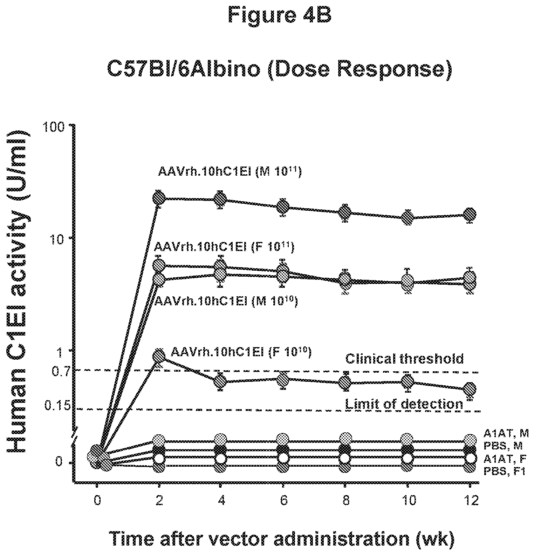

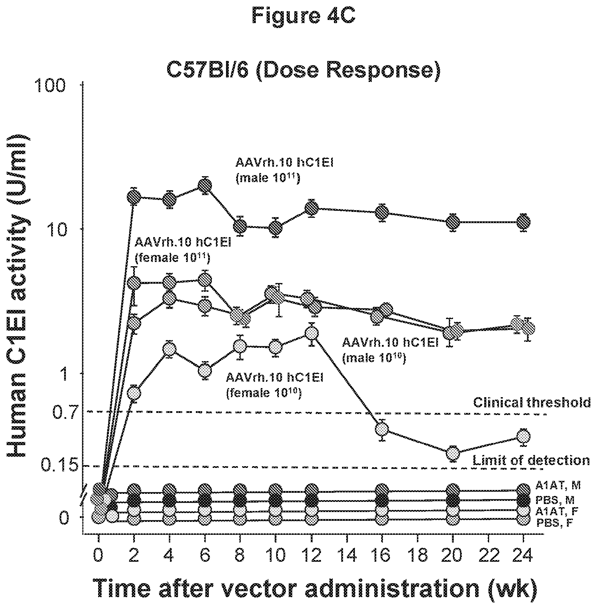

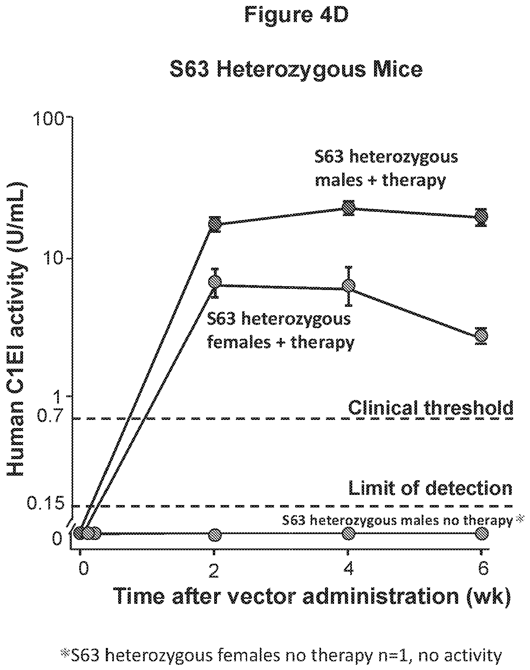

Figures 4B, 4C, and 4D are graphs of experimental data illustrating the dose dependent long term expression of human C1EI following single intravenous administration of 10¹⁰ gc AAVrh.10hC1EI 10¹¹ gc AAVrh.10hC1EI, 10¹¹ AAVrh.10hα1AT (control), or PBS to C57Bl/6Albino (Figure 4B), C57Bl/6 (Figure 4C) mice (n=4-5 mice/group), or S63 (Figure 4D) mice.

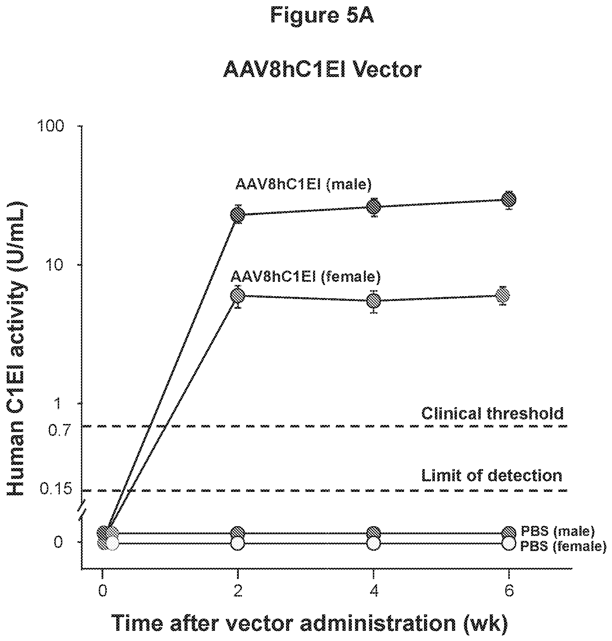

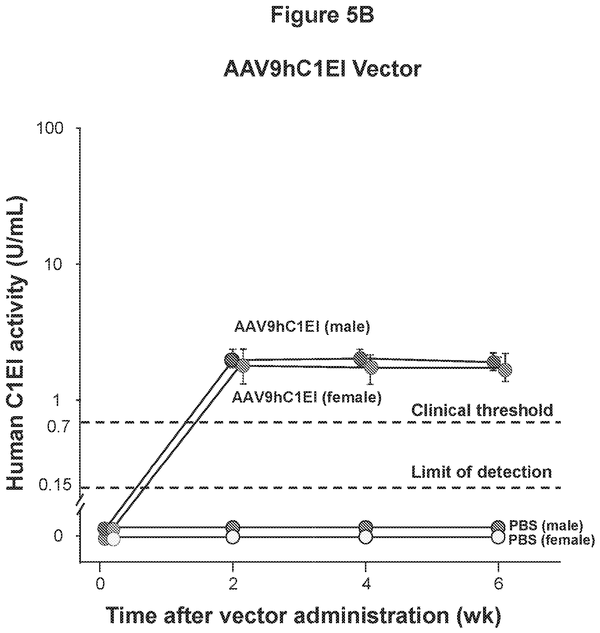

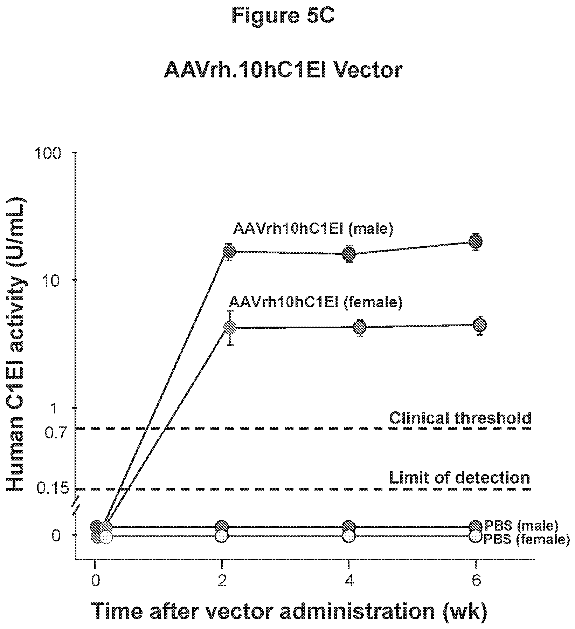

Figures 5A, 5B, and 5C are graphs of experimental data illustrating the long term expression of human C1EI following a single intravenous administration of 10¹¹ gc AAV8hC1EI (Figure 5A), AAV9hC1EI (Figure 5B), or AAVrh.10hC1EI (Figure 5C) to C57/Bl/6 mice (n=5 males/group, n=5 females/group, administration of PBS served as a control).

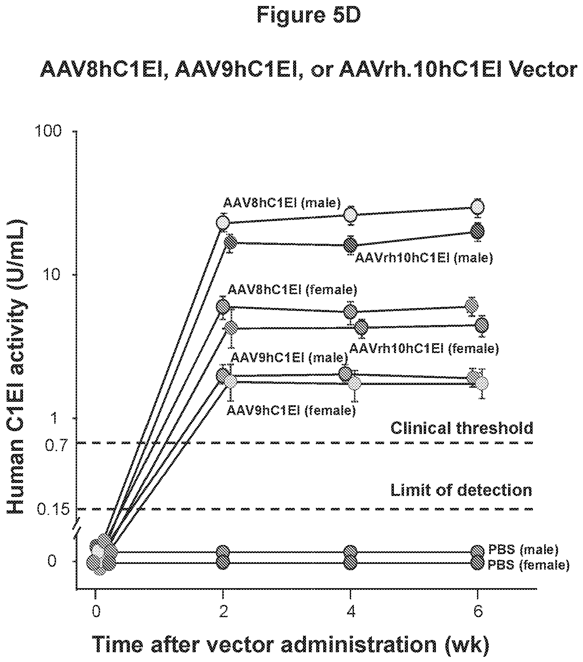

Figure 5D is a graph of the combined data from Figures 5A-5C.

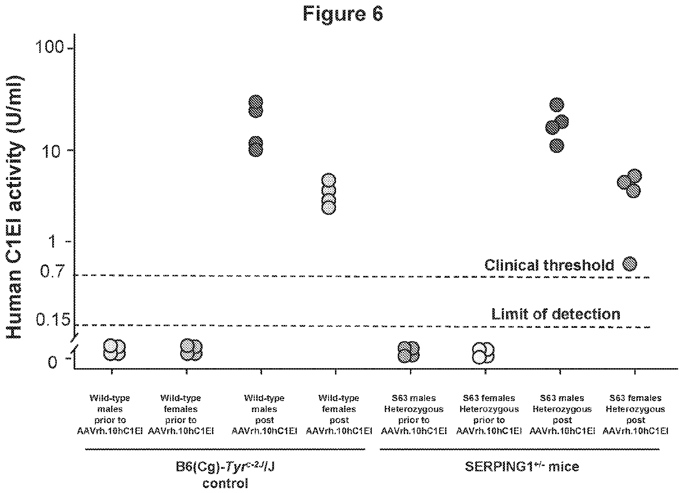

Figure 6 is a graph of experimental data illustrating the changes in human C1EI activity two weeks following a single administration of AAVrh.10hC1EI in S63 and control wild-type B6(Cg)-*Tyr^{c-2J}*/J mice (S63 mice: n=4 males/group, n=4 females/group; B6(Cg)-*Tyr^{c-2J}*/J mice: n=4 males/group, n=4 females/group).

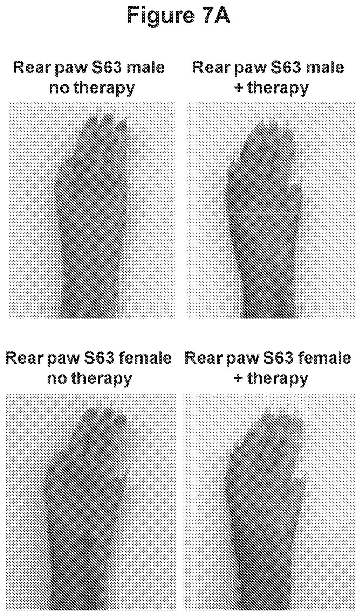

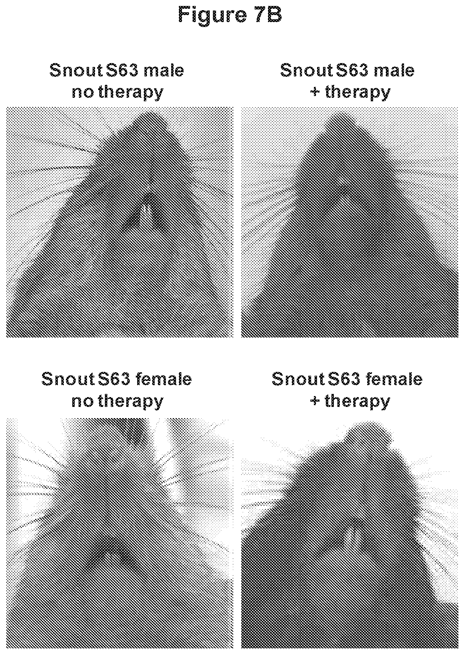

Figures 7A and 7B depict the effect of treatment of S63 SERPING1^{+/-} mice with AAVrh.10hC1EIL. Two weeks after S63 SERPING1^{+/-} mice were administered AAVrh.10hC1EI Evans blue dye was administered by tail vein injection and after 30 minutes mice were photographed. Figure 7A depicts the blue dye in the hind paws of S63 heterozygous untreated mice and S63 heterozygous AAVrh. 10hC1EI treated mice (n=4 male, n=4 female). Figure 7B depicts the blue dye in the snouts of S63 heterozygous untreated mice and S63 heterozygous AAVrh.10hC1EI treated mice (n=4 male, n=4 female).

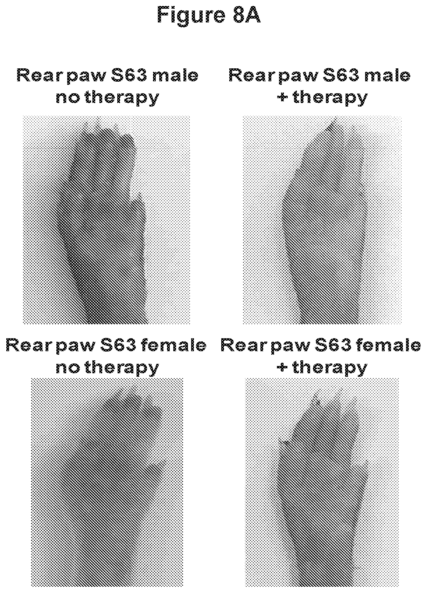

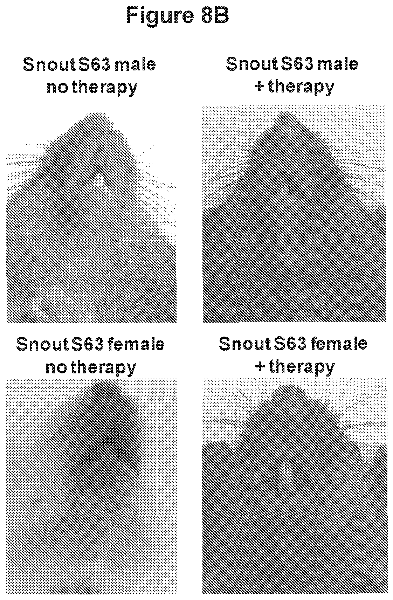

Figures 8A and 8B depict the effect of treatment of S63 SERPING1^{+/-} mice with AAVrh.10hC1EI. Six weeks after S63 SERPING1^{+/-} mice were administered AAVrh.10hC1EI Evans blue dye was administered by tail vein injection and after 30 minutes mice were photographed. Figure 8A depicts the blue dye in the hind paws of S63 heterozygous untreated mice and S63 heterozygous AAVrh. 10hC1EI treated mice (n=4 male, n=4 female). Figure 8B depicts the blue dye in the snouts of S63 heterozygous untreated mice and S63 heterozygous AAVrh.10hC1EI treated mice (n=4 male, n=4 female).

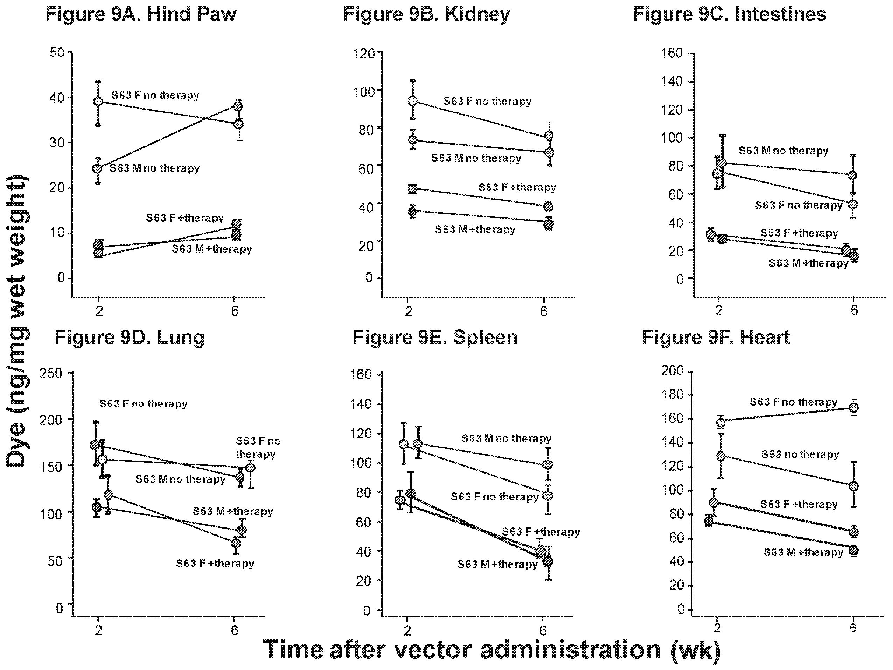

Figures 9A-9F are graphs of experimental results of the quantitative weight of dye of the vascular permeability response of SERPING1^{+/-} S63 AAVrh.10hC1EI treated and AAVrh.10hC1EI untreated mice (n=5/group) in the hind paw (Figure 9A), kidney (Figure 9B), intestines (Figure 9C), lung (Figure 9D), spleen (Figure 9E), and heart (Figure 9F).

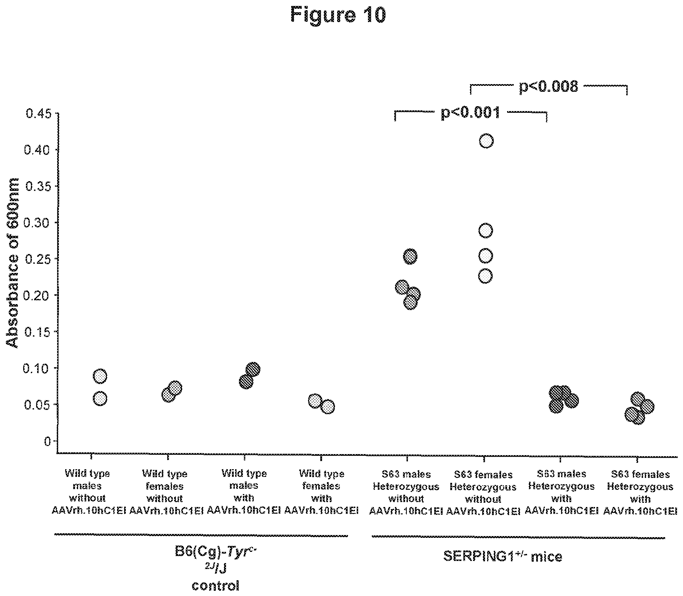

Figure 10 is a graph of experimental results showing the spectrophotometric analysis of the vascular permeability response of SERPING1^{+/-} S63 untreated mice (n=4 females, n=4 males) and S63 SERPING1^{+/-} AAVrh.10hC1EI treated mice (n=4 females, n=4 males). B6(Cg)-*Tyr^{c-2J}l*J wild-type treated and untreated mice served as controls (n=2 females, n=2 males).

### DETAILED DESCRIPTION OF THE INVENTION

The invention is predicated, at least in part, upon the ability of vectors to be safely administered to humans and to provide persistent expression of a therapeutic transgene. The invention provides a vector for use as defined in the claims wherein the vector comprises, consists essentially of, or consists of a promoter operably linked to a nucleic acid sequence that encodes human C1 esterase inhibitor (C1EI). When the inventive vector consists essentially of a promoter operably linked to a nucleic acid sequence that encodes human C1EI, additional components can be included that do not materially affect the vector (e.g., genetic elements such as poly(A) sequences or restriction enzyme sites that facilitate manipulation of the vector *in vitro*). When the vector consists of a promoter operably linked to a nucleic acid sequence that encodes human C1EI, the vector does not comprise any additional components (i.e., components that are not endogenous to the vector and are not required to effect expression of the nucleic acid sequence to thereby provide the protein).

The vector for use of the invention is an AAV vector.

Adeno-associated virus is a member of the *Parvoviridae* family and comprises a linear, single-stranded DNA genome of less than about 5,000 nucleotides. AAV requires co-infection with a helper virus (i.e., an adenovirus or a herpes virus), or expression of helper genes, for efficient replication. AAV vectors used for administration of therapeutic nucleic acids typically have approximately 96% of the parental genome deleted, such that only the terminal repeats (ITRs), which contain recognition signals for DNA replication and packaging, remain. This eliminates immunologic or toxic side effects due to expression of viral genes. In addition, delivering specific AAV proteins to producing cells enables integration of the AAV vector comprising AAV ITRs into a specific region of the cellular genome, if desired (see, e.g., U.S. Patents 6,342,390 and 6,821,511). Host cells comprising an integrated AAV genome show no change in cell growth or morphology (see, for example, U.S. Patent 4,797,368).

The AAV ITRs flank the unique coding nucleotide sequences for the non-structural replication (Rep) proteins and the structural capsid (Cap) proteins (also known as virion proteins (VPs)). The terminal 145 nucleotides are self-complementary and are organized so that an energetically stable intramolecular duplex forming a T-shaped hairpin may be formed. These hairpin structures function as an origin for viral DNA replication by serving as primers for the cellular DNA polymerase complex. The Rep genes encode the Rep proteins Rep78, Rep68, Rep52, and Rep40. Rep78 and Rep68 are transcribed from the p5 promoter, and Rep 52 and Rep40 are transcribed from the p19 promoter. The Rep78 and Rep68 proteins are multifunctional DNA binding proteins that perform helicase and nickase functions during productive replication to allow for the resolution of AAV termini (see, e.g., Im et al., Cell, 61: 447-57 (1990)). These proteins also regulate transcription from endogenous AAV promoters and promoters within helper viruses (see, e.g., Pereira et al., J. Virol., 71: 1079-1088 (1997)). The other Rep proteins modify the function of Rep78 and Rep68. The cap genes encode the capsid proteins VP1, VP2, and VP3. The cap genes are transcribed from the p40 promoter.

The AAV vector for use according to the invention can be generated using any AAV serotype known in the art. Several AAV serotypes and over 100 AAV variants have been isolated from adenovirus stocks or from human or nonhuman primate tissues (reviewed in, e.g., Wu et al., Molecular Therapy, 14(3): 316-327 (2006)). Generally, the AAV serotypes have genomic sequences of significant homology at the nucleic acid sequence and amino acid sequence levels, such that different serotypes have an identical set of genetic functions, produce virions which are essentially physically and functionally equivalent, and replicate and assemble by practically identical mechanisms. AAV serotypes 1-6 and 7-9 are defined as "true" serotypes, in that they do not efficiently cross-react with neutralizing sera specific for all other existing and characterized serotypes. In contrast, AAV serotypes 6, 10 (also referred to as Rh10), and 11 are considered "variant" serotypes as they do not adhere to the definition of a "true" serotype. AAV serotype 2 (AAV2) has been used extensively for gene therapy applications due to its lack of pathogenicity, wide range of infectivity, and ability to establish long-term transgene expression (see, e.g., Carter, B.J., Hum. Gene Ther., 16: 541-550 (2005); and Wu et al., *supra*). Genome sequences of various AAV serotypes and comparisons thereof are disclosed in, for example, GenBank Accession numbers U89790, J01901, AF043303, and AF085716; Chiorini et al., J. Virol., 71: 6823-33 (1997); Srivastava et al., J. Virol., 45: 555-64 (1983); Chiorini et al., J. Virol., 73: 1309-1319 (1999); Rutledge et al., J. Virol., 72: 309-319 (1998); and Wu et al., J. Virol., 74: 8635-47 (2000)).

AAV rep and ITR sequences are particularly conserved across most AAV serotypes. For example, the Rep78 proteins of AAV2, AAV3A, AAV3B, AAV4, and AAV6 are reportedly about 89-93% identical (see Bantel-Schaal et al., J. Virol., 73(2): 939-947 (1999)). It has been reported that AAV serotypes 2, 3A, 3B, and 6 share about 82% total nucleotide sequence identity at the genome level (Bantel-Schaal et al., *supra*). Moreover, the rep sequences and ITRs of many AAV serotypes are known to efficiently cross-complement (i.e., functionally substitute) corresponding sequences from other serotypes during production of AAV particles in mammalian cells.

Generally, the cap proteins, which determine the cellular tropicity of the AAV particle, and related cap protein-encoding sequences, are significantly less conserved than Rep genes across different AAV serotypes. In view of the ability of Rep and ITR sequences to cross-complement corresponding sequences of other serotypes, the AAV vector for use according to the invention comprises a mixture of serotypes and thereby be a "chimeric" or "pseudotyped" AAV vector. A chimeric AAV vector typically comprises AAV capsid proteins derived from two or more (e.g., 2, 3, 4, etc.) different AAV serotypes. In contrast, a pseudotyped AAV vector comprises one or more ITRs of one AAV serotype packaged into a capsid of another AAV serotype. Chimeric and pseudotyped AAV vectors are further described in, for example, U.S. Patent 6,723,551; Flotte, Mol. Ther., 13(1): 1-2 (2006); Gao et al., J. Virol., 78: 6381-6388 (2004); Gao et al., Proc. Natl. Acad. Sci. USA, 99: 11854-11859 (2002); De et al., Mol. Ther., 13: 67-76 (2006); and Gao et al., Mol. Ther., 13: 77-87 (2006).

In one embodiment, the AAV vector is generated using an AAV that infects humans (e.g., AAV2). In a preferred embodiment the AAV vector generated using an AAV that infects humans is AAV8 or AAV9. Alternatively, the AAV vector is generated using an AAV that infects non-human primates, such as, for example, the great apes (e.g., chimpanzees), Old World monkeys (e.g., macaques), and New World monkeys (e.g., marmosets). Preferably, the AAV vector is generated using an AAV that infects a non-human primate pseudotyped with an AAV that infects humans. Examples of such pseudotyped AAV vectors are disclosed in, e.g., Cearley et al., Molecular Therapy, 13: 528-537 (2006). In one embodiment, an AAV vector can be generated which comprises a capsid protein from an AAV that infects rhesus macaques pseudotyped with AAV2 inverted terminal repeats (ITRs). In a particularly preferred embodiment, the inventive AAV vector comprises a capsid protein from AAV10 (also referred to as "AAVrh.10"), which infects rhesus macaques pseudotyped with AAV2 ITRs (see, e.g., Watanabe et al., Gene Ther., 17(8): 1042-1051 (2010); and Mao et al., Hum. Gene Therapy, 22: 1525-1535 (2011)).

The vector for use according to the invention comprises a promoter operably linked to a nucleic acid sequence that encodes human C1EI. DNA regions are "operably linked" when they are functionally related to each other. A promoter is "operably linked" to a coding sequence if it controls the transcription of the sequence.

A "promoter" is a region of DNA that initiates transcription of a particular gene. A large number of promoters from a variety of different sources are well known in the art. Representative sources of promoters include for example, virus, mammal, insect, plant, yeast, and bacteria, and suitable promoters from these sources are readily available, or can be made synthetically, based on sequences publicly available, for example, from depositories such as the ATCC as well as other commercial or individual sources. Promoters can be unidirectional (i.e., initiate transcription in one direction) or bi-directional (i.e., initiate transcription in either a 3' or 5' direction).

The promoter of the inventive vector can comprise, consist essentially of, or consist of any promoter known in the art. Examples of classes of such promoters include constitutively active promoters (e.g., human beta-actin, chicken beta-actin, cytomegalovirus (CMV), and SV40), cell type specific promoters (e.g., CD19 gene promoter, CaMKIIa, and UAS), or an inducible promoter (e.g., the Tet system (U.S. Patents 5,464,758 and 5,814,618), the Ecdysone inducible system (No et al., Proc. Natl. Acad. Sci., 93: 3346-3351 (1996)), the T-REX^{™} system (Invitrogen, Carlsbad, CA), the Cre-ERT tamoxifen inducible recombinase system (Indra et al., Nuc. Acid. Res., 27: 4324-4327 (1999); Nuc. Acid. Res., 28: e99 (2000); U.S. Patent 7,112,715; and Kramer & Fussenegger, Methods Mol. Biol., 308: 123-144 (2005)), and the LACSWITCH^{™} System (Stratagene, San Diego, CA)).

In a preferred embodiment of the invention the promoter is a constitutively active promoter, an inducible promoter, or a cell-type specific promoter. In a more preferred embodiment of the invention the promoter is a constitutively active promoter, and preferably the constitutively active promoter is the chicken beta-actin promoter.

"Nucleic acid sequence" is intended to encompass a polymer of DNA or RNA, i.e., a polynucleotide, which can be single-stranded or double-stranded and which can contain non-natural or altered nucleotides. The terms "nucleic acid" and "polynucleotide" as used herein refer to a polymeric form of nucleotides of any length, either ribonucleotides (RNA) or deoxyribonucleotides (DNA). These terms refer to the primary structure of the molecule, and thus include double- and single-stranded DNA, and double- and single-stranded RNA. The terms include, as equivalents, analogs of either RNA or DNA made from nucleotide analogs and modified polynucleotides such as, though not limited to, methylated and/or capped polynucleotides.

The nucleic acid sequence operably linked to the promoter of the inventive vector may comprise any nucleic acid sequence that encodes a therapeutic gene which inhibits or reduces submucosal or subcutaneous edema in a mammal with hereditary angioedema. The nucleic acid sequence encodes human C1EI. Preferably the nucleic acid sequence encodes for the full length human C1EI protein; however, the nucleic acid sequence may also encode variants, such as truncations, as long as the protein produced maintains the functional characteristics of the full length protein (e.g., inhibition of the complement system). The nucleic acid sequence may also encode for fusion proteins which are comprised of an active protein human C1EI and a second moiety, usually a protein, which improves the properties (e.g., efficacy, solubility, or half-life) of the active protein. Examples of the second moiety are known in the art and include, for example, the Fc domain of an immunoglobulin and polyethylene glycol (PEG).

C1EI is a protease inhibitor that circulates in the plasma at levels around 21-40 mg/kg. One of the main functions of C1EI is the inhibition of the complement system to prevent spontaneous activation by binding to and inactivating the C1r and C1s proteases of the classical complement pathway. Human C1EI comprises a heavily glycosylated single-chain polypeptide of 500 amino acid residues and is normally produced by hepatocytes, fibroblasts, monocytes, and endothelial cells. Human C1EI is encoded by the SERPING1 gene which is located on chromosome 11 at 11q11-q13.1. Chromosome 11q11-q13.1 is roughly 1.5 kilobases long and encompasses 8 coding exons. The amino acid sequence and nucleic acid sequence of human C1EI as, well as function variants of human C1EI, are well known in the art. An example of the amino acid sequence of a C1EI protein and nucleotide sequence encoding a C1E1 protein can be found at, for example, GenBank Accession number: AAM21515.1 (amino acid) (SEQ ID NO: 1) and AF435921.1 (nucleic acid) (SEQ ID NO: 2).

The nucleic acid sequence encoding the human C1EI, can be generated using methods known in the art. For example, nucleic acid sequences, polypeptides, and proteins can be recombinantly produced using standard recombinant DNA methodology (see, e.g., Sambrook et al., Molecular Cloning: A Laboratory Manual, 3rd ed., Cold Spring Harbor Press, Cold Spring Harbor, NY, 2001; and Ausubel et al., Current Protocols in Molecular Biology, Greene Publishing Associates and John Wiley & Sons, NY, 1994). Further, a synthetically produced nucleic acid sequence encoding human C1EI, can be isolated and/or purified from a source, such as a bacterium, an insect, or a mammal, e.g., a rat, a human, etc. Methods of isolation and purification are well-known in the art. Alternatively, the nucleic acid sequences described herein can be commercially synthesized. In this respect, the nucleic acid sequence can be synthetic, recombinant, isolated, and/or purified. The sequences (e.g., SEQ ID NOs: 1-2) can further be optimized for increased mRNA stability and to reduce the possibility of trans-inhibition by the mutant mRNA.

In addition to the promoter operably linked to a nucleic acid sequence encoding human C1EI, the vector preferably comprises additional expression control sequences, such as enhancers, polyadenylation signals, transcription terminators, internal ribosome entry sites (IRES), and the like, that provide for the expression of the nucleic acid sequence in a host cell. Exemplary expression control sequences are known in the art and described in, for example, Goeddel, Gene Expression Technology: Methods in Enzymology, Vol. 185, Academic Press, San Diego, CA. (1990).

The term "enhancer" as used herein, refers to a DNA sequence that increases transcription of, for example, a nucleic acid sequence to which it is operably linked. Enhancers can be located many kilobases away from the coding region of the nucleic acid sequence and can mediate the binding of regulatory factors, patterns of DNA methylation, or changes in DNA structure. A large number of enhancers from a variety of different sources are well known in the art and are available as or within cloned polynucleotides (from, e.g., depositories such as the ATCC as well as other commercial or individual sources). A number of polynucleotides comprising promoters (such as the commonly-used CMV promoter) also comprise enhancer sequences. Enhancers can be located upstream, within, or downstream of coding sequences. The nucleic acid sequence encoding the human C1EI may be operably linked to a CMV enhancer/chicken β-actin promoter (also referred to as a "CAG promoter") (see, e.g., Niwa et al., Gene, 108: 193-199 (1991); Daly et al., Proc. Natl. Acad. Sci. U.S.A., 96: 2296-2300 (1999); and Sondhi et al., Mol. Ther., 15: 481-491 (2007)).

The invention provides a composition for use according to the invention comprising, consisting essentially of, or consisting of the above-described vector and a pharmaceutically acceptable (e.g. physiologically acceptable) carrier. When the composition consists essentially of the inventive vector and a pharmaceutically acceptable carrier, additional components can be included that do not materially affect the composition (e.g., adjuvants, buffers, stabilizers, antiinflammatory agents, solubilizers, preservatives, etc.). When the composition consists of the inventive vector and the pharmaceutically acceptable carrier, the composition does not comprise any additional components. Any suitable carrier can be used within the context of the invention, and such carriers are well known in the art. The choice of carrier will be determined, in part, by the particular site to which the composition may be administered and the particular method used to administer the composition. The composition optionally can be sterile with the exception of the vector described herein. The composition can be frozen or lyophilized for storage and reconstituted in a suitable sterile carrier prior to use. The compositions can be generated in accordance with conventional techniques described in, e.g., Remington: The Science and Practice of Pharmacy, 21st Edition, Lippincott Williams & Wilkins, Philadelphia, PA (2001).

Suitable formulations for the composition include aqueous and non-aqueous solutions, isotonic sterile solutions, which can contain anti-oxidants, buffers, and bacteriostats, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid carrier, for example, water, immediately prior to use. Extemporaneous solutions and suspensions can be prepared from sterile powders, granules, and tablets of the kind previously described. Preferably, the carrier is a buffered saline solution. More preferably, the inventive vector is administered in a composition formulated to protect the inventive vector from damage prior to administration. For example, the composition can be formulated to reduce loss of the vector on devices used to prepare, store, or administer the vector, such as glassware, syringes, or needles. The composition can be formulated to decrease the light sensitivity and/or temperature sensitivity of the vector. To this end, the composition preferably comprises a pharmaceutically acceptable liquid carrier, such as, for example, those described above, and a stabilizing agent selected from the group consisting of polysorbate 80, L-arginine, polyvinylpyrrolidone, trehalose, and combinations thereof. Use of such a composition will extend the shelf life of the vector, facilitate administration, and increase the efficiency of the inventive method. Formulations for vector-containing compositions are further described in, for example, Wright et al., Curr. Opin. Drug Discov. Devel., 6(2): 174-178 (2003) and Wright et al., Molecular Therapy, 12: 171-178 (2005))

The composition also can be formulated to enhance transduction efficiency. In addition, one of ordinary skill in the art will appreciate that the inventive vector can be present in a composition with other therapeutic or biologically-active agents. For example, factors that control inflammation, such as ibuprofen or steroids, can be part of the composition to reduce swelling and inflammation associated with *in vivo* administration of the vector. Antibiotics, i.e., microbicides and fungicides, can be present to treat existing infection and/or reduce the risk of future infection, such as infection associated with gene transfer procedures.

The invention provides a vector for use in a a method of preventing or treating hereditary angioedema in a mammal wherein the inventive vector is administered to the mammal, whereupon the nucleic is expressed to produce the protein that prevents or treats hereditary angioedema. In a preferred embodiment the mammal is a human.

Preventing or treating hereditary angioedema encompasses any degree of amelioration of any physiological response or symptom brought on by hereditary angioedema.

Any route of administration can be used to deliver the composition to the mammal. Indeed, although more than one route can be used to administer the composition, a particular route can provide a more immediate and more effective reaction than another route. Preferably, the composition is administered via intramuscular injection. A dose of composition also can be applied or instilled into body cavities, absorbed through the skin (e.g., via a transdermal patch), inhaled, ingested, topically applied to tissue, or administered parenterally via, for instance, intravenous, intraperitoneal, intraoral, intradermal, subcutaneous, or intraarterial administration.

The composition can be administered in or on a device that allows controlled or sustained release, such as a sponge, biocompatible meshwork, mechanical reservoir, or mechanical implant. Implants (see, e.g., U.S. Patent 5,443,505), devices (see, e.g., U.S. Patent 4,863,457), such as an implantable device, e.g., a mechanical reservoir or an implant or a device comprised of a polymeric composition, are particularly useful for administration of the AAV vector. The composition also can be administered in the form of sustained-release formulations (see, e.g., U.S. Patent 5,378,475) comprising, for example, gel foam, hyaluronic acid, gelatin, chondroitin sulfate, a polyphosphoester, such as bis-2-hydroxyethyl-terephthalate (BHET), and/or a polylactic-glycolic acid.

The dose of the vector in the composition administered to the mammal will depend on a number of factors, including the size (mass) of the mammal, the extent of any side-effects, the particular route of administration, and the like. Preferably, in the use according to the invention a "therapeutically effective amount" of the composition comprising the inventive vector described herein is administered. A "therapeutically effective amount" refers to an amount effective, at dosages and for periods of time necessary, to achieve a desired therapeutic result. The therapeutically effective amount may vary according to factors such as the degree of allergen sensitivity, age, sex, and weight of the individual, and the ability of the vector to elicit a desired response in the individual.

In another embodiment, in the use according to the invention a "prophylactically effective amount" of the composition comprising the inventive vector is administered. A "prophylactically effective amount" refers to an amount effective, at dosages and for periods of time necessary, to achieve a desired prophylactic result (e.g., prevention of an immune response or allergic reaction). Subjects that are in need of prophylactic administration can be readily determined by routine testing known in the art. Additionally, subjects with a previous hereditary angioedema attack can be treated prophylactically against future attacks.

In a preferred embodiment of the invention, the composition is administered once to the mammal. It is believed that a single administration of the composition will result in persistent expression of human C1EI in the mammal with minimal side effects. However, in certain cases, it may be appropriate to administer the composition multiple times during a therapeutic or prophylactic treatment period and/or employ multiple administration routes, e.g., intramuscular and subcutaneous, to ensure sufficient exposure of cells to the composition. For example, the composition may be administered to the mammal two or more times (e.g., 2, 3, 4, 5, 6, 6, 8, 9, or 10 or more times) during a therapeutic or prophylactic treatment period.

The dose of vector in the composition required to achieve a particular therapeutic or prophylactic effect (i.e., reduction or inhibition of an allergic reaction) typically is administered in units of vector genome copies per cell (gc/cell) or vector genome copies/per kilogram of body weight (gc/kg). One of ordinary skill in the art can readily determine an appropriate vector dose range to treat a patient having a particular immune response based on these and other factors that are well known in the art.

The following examples further illustrate the invention but, of course, should not be construed as in any way limiting its scope.

### EXAMPLE 1

This example demonstrates the development and characterization of a C1EI deficient mouse model of hereditary angioedema.

The C1EI deficient mouse model was developed using the clustered regularly interspaced short palindromic repeat (CRISPR) and CRISPR-associated endonuclease 9 (Cas9) technology targeting exon 3 (E3) of SERPING1. The Cas9 endonuclease is directed to generate site-specific DNA double-stranded breaks (DSB) when provided with a synthetic single-guide RNA (sgRNA) targeting the desired sequence. In mammalian cells, the DSB produced by the Cas9 cleavage are repaired by the cell non-homologous end joining (NHEJ) repair pathway that introduces insertion/deletion mutations (InDels) in the gene, which in turn cause frameshifts resulting in null alleles. We leveraged this technology to introduce targeted null mutations in the mouse SERPING1 gene.

F1 zygotes from CBA/J x B6/J mice were co-injected with a functional single guide RNA targeting exon 3 of SERPING1 and Cas9 mRNA. Embryos were implanted at the 2-cell stage into surrogate mothers and all pups were screened for presence of SERPING1 exon 3 insertion / deletions by T7 endonuclease I-based PCR analysis. Mice with InDels in exon 3 were selected for breeding and further DNA sequencing. Mice were bred as pairs (one female with one male) or trios (two females with one male); with B6(Cg)-*Tyr*^{*c*-*2J*}/J mice (B6 Albino mice, Jackson Laboratory, Bar Harbor, ME). All mice were housed in microisolator cages and maintained according to standard guidelines.

All first generation pups were screened for exon 3 mutations by T7 endonuclease I digestion. Briefly, mouse genomic DNA was extracted from tail tissue (0.5 cm, tail tip) and exon 3 was amplified by PCR using primers flanking the mutation target region (forward primer, 5'-TTGCACGGCGGTCACTGGACACAGATAACT-3'; reverse primer, 5'-CAAGCGGCTCCGGGCAGAAAGGGTTCA-3'). PCR products were then denatured at high temperature, re-annealed for DNA duplex formation, and digested using T7 endonuclease I (T7E-I), which cleaves mismatched DNA duplexes (heteroduplexes). Digestion by T7EI of mutation carrying heteroduplexes results in two or more smaller DNA fragments that can be resolved by agarose gel electrophoresis.

Founder heterozygous mice carrying mutations in exon 3 were selected for genomic DNA sequencing. Tail tip tissue (0.5 cm) was obtained from SERPING1 knockout mice anesthetized under isoflurane gas, and genomic DNA (gDNA) extracted using KAPA Express Extract DNA Extraction Kit (KAPA Biosytems; Wilmington, MA). Genomic DNA was amplified using specific primers that flanked the targeted exon 3 region of the SERPING1 gene (forward primer, 5'-TTGCACGGCGGTCACTGGACACAGATAACT-3'; reverse primer, 5'-CAAGCGGCTCCGGGCAGAAAGGGTTCA-3'). PCR amplification was performed with *Taq* polymerase and reagents supplied by KAPA2G Fast (HotStart) Genotyping Mix (KAPA Biosytems; Wilmington, MA). Each cycle of denaturation (95°C, 30 sec), annealing (60°C, 30 sec), and extension (72°C, 30 sec) was repeated 35 times. The resultant PCR product was purified using QIAGEN PCR Purification Kit (QIAGEN; Valencia, CA), the DNA eluted in 50 µl buffer EB, and cloned into a TOPO vector for DNA sequencing (Life Technologies, Norwalk, CT). Multiple TOPO clones for each mutant were sequenced by Sanger technology to identify the mutation introduced by the CRISPR/Cas9-directed NHEJ DNA repair system.

Using this technology, we have generated a number of mice with different genetic variants in exon 3 of the SERPING1 gene (Figure 1). One of these mice (referred to as "S63") has been characterized in detail and used as a model of hereditary angioedema for the demonstration of efficacy of the AAVrh. 10hC1EI therapy. The S63 mice appeared normal at birth, and subsequently developed and bred normally.

Murine C1EI (mC1EI) levels were evaluated in the S63 mouse sera by ELISA (Biomatik, Wilmington, DE) according to manufacturer's instructions. Murine bradykinin levels were evaluated in the S63 mouse sera by ELISA (MyBioSource, San Diego, CA) according to manufacturer's instructions. Human C1EI (hC1EI) activity levels were evaluated by a chromogenic activity assay that measures the ability of the protein to inhibit its natural substrate, C1 esterase (TECHNOCHROM^{®} C1-INH [CE], DiaPharma Group, West Chester, OH). In brief, the assay is based on the inhibition of C1 esterase activity. C1 esterase cleavage of substrate C1-1(C2H5CO-lys (ε-Cbo)-Gly-Arg-pNA) releases the chromophore para-nitroaniline (pNA). The absorbance of released pNA is measured at OD 405 nm and is inversely proportional to the concentration (activity) of C1 esterase inhibitor present in the serum or plasma. The activity assay was conducted per the manufacturer's protocol, and results expressed as a unit of function (U/mL). All analyses were performed in duplicate. Evans blue dye (30 mg/kg in 100 µl phosphate buffered saline; Sigma Chemical Co., St. Louis, MO) was injected into the tail vein of 6 to 8 weeks old mice. Photographs of hind-paws and snouts were taken 30 min after injection of Evans blue dye. After the mice were euthanized by CO2 inhalation, paws were removed, blotted dry, and weighed. The Evans blue dye was extracted (equal weights) with 1 ml of formamide overnight at 55°C and measured spectrophotometrically at 600 nm.

The results from these studies indicate that the level of C1EI in the S63 mice was markedly lower than the C1EI level in the wild-type controls (Figure 2A) and bradykinin levels were elevated in S63 mice compared to wild-type controls (Figure 2B). Indicating that the S63 mouse model provides an *in vivo* animal model of hereditary angioedema.

### EXAMPLE 2

This example demonstrates the design and *in vitro* characterization of the AAV-vector comprising a promoter operably linked to a nucleic acid sequence encoding human C1EI.

The expression cassette consists of the AAV2 inverted terminal repeats (ITR), encapsidation signal (ψ), cytomegalovirus (CMV) enhancer chicken-β-actin promoter (CAG promoter) operably linked to human C1EI cDNA sequence and the rabbit β-globin polyadenylation signal (Figure 3A). The hC1EI cDNA sequence was optimized for increased mRNA stability and to reduce the possibility of trans-inhibition by the mutant mRNA. hC1EI cDNA was sequence-optimized using human-biased codons and removal of: mRNA instability elements; low (<30%) or rich (>80%) GC regions; translation initiation sequences within the coding region; and potential splicing signals. Optimized hC1EI cDNA was synthesized with an optimal Kozak consensus.

The optimized full length human C1EI cDNA sequence was synthesized and cloned into the pAAV plasmid-under control of the CAG promoter. The AAV-hC1EI plasmid was produced by co-transfection into human embryonic kidney 293T cells (HEK 293T; American Type Culture Collection) of the pAAV plasmid together with a plasmid carrying the AAV Rep proteins derived from AAV2 needed for vector replication, the AAVrh.10 viral structural (Cap) proteins VP1, 2 and 3, which define the serotype of the produced AAV vector; and the adenovirus helper functions of E2, E4 and VA RNA. The AAV-hC1EI vector (referred to as "AAVrh.10hC1EI') was purified by iodixanol gradient and QHP anion exchange chromatography. Vector genome titers were determined by quantitative TaqMan real-time PCR analysis. A vector coding for an irrelevant protein, AAV-GFP was used as control for the expression studies.

To assess AAVr.10hC1EI directed expression of the human C1EI protein *in vitro,* HEK 293T cells were transfected with the AAV-hC1EI plasmid or the control plasmid, and supernatant was harvested 72 hr later. Human C1EI expression in supernatant was evaluated by Coomassie blue stain SDS-PAGE and Western analysis with peroxidase-conjugated goat anti-human kappa light-chain antibody and peroxidase-conjugated anti-human C1EI antibody. As shown in Figure 3B, human C1EI was detected in cell culture supernatants. The results from this example show the expression of C1EI from an AAV vector.

### EXAMPLE 3

This example demonstrates long term *in vivo* expression ofAAVrh. 10hC1EI in wild type mice.

To evaluate long-term *in vivo* serum expression of human C1EI after treatment with the AAVrh.10hC1EI vector, C57Bl/6Albino, C57Bl/6, or SERPING^{+/-} S63 male and female mice at age 6-8 weeks received a single administration of the AAVrh.10hC1EI vector, control AAVrh. 10hα1AT vector, or phosphate buffered saline (PBS) at 10¹⁰ or 10¹¹ genome copies (gc) by intravenous injection in 100 µl volume. Blood from the tail vein was assessed at time 0 and at various time points in the mice until week 20. Blood samples were allowed to clot for 1 hour at 23 °C followed by centrifugation at 13,000 RPM for 10 minutes to collect the serum. Activity of human C1EI was analyzed in each sample.

To evaluate long-term *in vivo* serum expression of human C1EI in after treatment with the AAV8hC1EI vector, the AAV9hC1EI vector, or the AAVrh.10hC1EI vector, C57Bl/6 male and female wild-type mice at age 6-8 weeks received a single administration of 10¹¹ genome copies (gc) of either the AAV8hC1EI vector, the AAV9hC1EI vector, the AAVrh.10hC1EI vector, or phosphate buffered saline (PBS) by intravenous injection in 100 µl volume. Blood from the tail vein was assessed at time 0 and at various time points in the mice until week 6. Blood samples were allowed to clot for 1 hour at 23 °C followed by centrifugation at 13,000 RPM for 10 minutes to collect the serum. Activity of human C1EI was analyzed in each sample.

As shown in Figure 4A, expression of human C1EI was demonstrated for the duration of the experiment (20 weeks), and the activity level of hC1EI was greater than the clinical threshold value at 20 weeks after vector administration (n=5 males/group; n=5 females/group). Additionally, as shown in Figures 4B and 4C a dose dependent expression of human C1EI was demonstrated for the duration of the experiment (12 weeks for Figure 4B and 24 weeks for Figure 4C), and the activity level of hC1EI was greater than the clinical threshold value in male mice treated with 10¹¹ or 10¹⁰gc AAVrh.10hC1EI and female mice treated with 10¹¹ gc AAVrh.10hC1EI for the duration of the respective experiments (n=5 males/group; n=5 females/group). As shown in Figure 4D, expression of human C1EI was demonstrated for the duration of the experiment (6 weeks), and the activity level of hC1EI was greater than the clinical threshold value at 6 weeks after vector administration (AAVrh.10hC1EI treated n=3mice/group; No therapy (PBS control) n=3 males and 1 female).

As shown in Figures 5A-5C expression of human C1EI was demonstrated for the duration of the experiment (6 weeks), and the activity level of hC1EI was greater than the clinical threshold value at 6 weeks after either AAV8hC1EI, AAV9hC1EI, or AAVrh.10hC1EI vector administration (n=5 males/group; n=5 females/group). Additionally, as shown in Figure 5D the activity of human C1EI was similar for each of the vectors tested.

These data demonstrate that each of the AAV8hC1EI, AAV9hC1EI, and AAVrh. 10hC1EI vector can provide long-term human C1EI expression from a single administration.

### EXAMPLE 4

This example demonstrates the treatment of hereditary angioedema by administering the AAVrh.10hC1EI in the mouse model of hereditary angioedema.

To assess AAVrh.10hC1EI directed expression of hC1EI protein *in vivo,* S63 (SERPING1^{+/-} ) mice, age 6 to 8 weeks, were administered a one-time dose of AAVrh. 10hC1EI at 10¹¹ genome copies (gc). Injections were performed intravenously in 100 µl volumes. Blood (100 µl) from the tail vein was assessed at 2 weeks after vector administration. Blood samples were allowed to clot for 1 hr, 23°C, followed by centrifugation at 13,000 RPM for 10 min to collect serum. Activity of hC1EI was measured at 2 weeks in n=4 males and n= 4 females. Vector treated (AAVrh.10hC1EI at 10¹¹ gc) and untreated wild-type B6(Cg)-*Tyr^{c-2J}*/J (Jackson Labs) mice, n=4 males and n= 4 females served as controls. At 2 and 6 weeks after vector administration, the mice were administered Evans blue dye (30 mg/kg in 100 µl phosphate buffered saline) by tail vein injection. Photographs of snouts and hind paws were taken 30 min after injection of the Evans blue dye. After the mice were euthanized by CO₂ inhalation, hind-paws were removed, blotted dry, and weighed. The Evans blue dye was extracted from equal weights of hind-paws, kidney, intestines, lung, spleen, and heart with 1 ml of formamide overnight at 55°C and measured spectrophotometrically at 600 nm. Vector treated (AAVrh. 10hC1EI at 10¹¹ gc) and untreated B6(Cg)-*Tyr^{c-2J}*/J (Jackson Labs) mice, served as controls.

The results from these studies indicated that hC1EI activity 2 weeks after AAVrh.10hC1EI gene transfer was greater than the clinical threshold normal value. The same levels of hC1EI activity were observed in B6(Cg)-*Tyr^{c-2J}*/J control mice. No human C1EI activity was detected in serum from mice that were untreated (Figure 6). Untreated S63 male and female mice had markedly increased vascular permeability compared with the wild-type mice. The blue coloration of the snout and hind-paws was observed within minutes after the Evans Blue dye injection and was much more intense in S63 mice than in the wild-type mice at 2 weeks post vector administration (Figure 7A and 7B). Similarly, the blue coloration of the snout and hind-paws was observed within minutes after the Evans Blue dye injection and was much more intense in S63 mice than in the wild-type mice at 6 weeks post vector administration (Figure 8A and 8B). Non-treated S63 mice visually exhibited greater extravasation of dye in their hind paws and snouts when compared to the AAVrh.10hC1EI treated group. Dye extravasation was comparable in B6(Cg)-*Tyr^{c-2J}*/J wild-type treated and untreated mice. The quantitative leak phenotype of treated and untreated S63 SERPING1 heterozygous mice was determined by measuring the dye extravasation in major organs (Figures 9A-9F). The observed phenotype was validated by spectrophotometric analysis of extracted dye from hind-paws (Figure 10). Treated mice had significantly (p<0.001 males, p<0.008 females) lower levels of dye extravasation than their non-treated littermates. These results indicate that treatment of hereditary angioedema with the AAVrh.10hC1EI vector results in a marked reduction of the symptoms of hereditary angioedema.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description.

## Claims

1. A chimeric or pseudotyped AAV vector comprising a promoter operably linked to a nucleic acid sequence which encodes human C1 esterase inhibitor (C1EI), optionally in a composition, for use in treating or preventing hereditary angioedema in a mammal.

2. The AAV vector for use of claim 1, wherein the AAV vector is administered to the mammal once before and/or after onset of edema.

3. The AAV vector for use of claim 2, wherein the AAV vector is administered to the mammal two or more times before and/or after onset of edema.

4. The AAV vector for use of any one of claims 1-3, wherein the mammal is a human.

5. The AAV vector for use of any one of claims 1-4, wherein AAV vector is a non-human adeno-associated virus.

6. The AAV vector for use of claim 5, wherein the non-human adeno-associated virus is a rhesus macaque adeno-associated virus, optionally an adeno-associated virus serotype rh.10.

7. The AAV vector for use of any of claims 1-6, wherein the promoter is a cell type specific promoter, an inducible promoter, or a constitutively active promoter, optionally a cell-specific promoter, an inducible promoter, or a chicken-beta actin promoter.

8. The AAV vector for use of claim 7, wherein the promoter is a chicken-beta actin promoter, and the AAV vector further comprises a cytomegalovirus (CMV) enhancer sequence.

9. The AAV vector for use of any of claims 1-8, wherein the AAV vector comprises a nucleic acid sequence which encodes C1EI comprising SEQ ID NO: 1.

10. The AAV vector for use of any of claims 1-9, wherein the AAV vector is administered to the mammal prophylactically.

11. The AAV vector for use of any one of claims 1-10, wherein the AAV vector is in a composition formulated for intraoral, intramuscular, transdermal, intravenous, intraarterial, subcutaneous, intradermal, or intraperitoneal administration.

## Patentansprüche

1. Chimärer oder pseudotypisierter AAV-Vektor, der einen Promotor umfasst, der funktionell mit einer Nukleinsäuresequenz verbunden ist, die den humanen C1-Esterase-Inhibitor (C1EI) codiert, optional in einer Zusammensetzung, zur Verwendung bei der Behandlung oder Vorbeugung des hereditären Angioödems bei einem Säugetier.

2. AAV-Vektor zur Verwendung nach Anspruch 1, wobei der AAV-Vektor dem Säugetier einmal vor und/oder nach dem Auftreten eines Ödems verabreicht wird.

3. AAV-Vektor zur Verwendung nach Anspruch 2, wobei der AAV-Vektor dem Säugetier zweimal oder mehrmals vor und/oder nach dem Auftreten eines Ödems verabreicht wird.

4. AAV-Vektor zur Verwendung nach einem der Ansprüche 1 bis 3, wobei das Säugetier ein Mensch ist.

5. AAV-Vektor zur Verwendung nach einem der Ansprüche 1 bis 4, wobei der AAV-Vektor ein nicht-humanes Adeno-assoziiertes Virus ist.

6. AAV-Vektor zur Verwendung nach Anspruch 5, wobei das nicht-humane Adeno-assoziierte Virus ein Rhesusaffen-Adeno-assoziiertes Virus ist, optional ein Adeno-assoziiertes Virus vom Serotyp rh.10.

7. AAV-Vektor zur Verwendung nach einem der Ansprüche 1 bis 6, wobei der Promotor ein zelltypspezifischer Promotor, ein induzierbarer Promotor oder ein konstitutiv aktiver Promotor ist, optional ein zellspezifischer Promotor, ein induzierbarer Promotor oder ein Hühner-Beta-Aktin-Promotor.

8. AAV-Vektor zur Verwendung nach Anspruch 7, wobei der Promotor ein Hühner-Beta-Aktin-Promotor ist und der AAV-Vektor ferner eine Cytomegalovirus (CMV)-Enhancer-Sequenz umfasst.

9. AAV-Vektor zur Verwendung nach einem der Ansprüche 1 bis 8, wobei der AAV-Vektor eine Nukleinsäuresequenz umfasst, die C1EI codiert, die SEQ ID NO: 1 umfasst.

10. AAV-Vektor zur Verwendung nach einem der Ansprüche 1 bis 9, wobei der AAV-Vektor dem Säugetier prophylaktisch verabreicht wird.

11. AAV-Vektor zur Verwendung nach einem der Ansprüche 1 bis 10, wobei der AAV-Vektor in einer Zusammensetzung vorliegt, die für die intraorale, intramuskuläre, transdermale, intravenöse, intraarterielle, subkutane, intradermale oder intraperitoneale Verabreichung formuliert ist.

## Revendications

1. Vecteur AAV chimérique ou pseudotypé comprenant un promoteur lié de manière opérationnelle à une séquence d'acides nucléiques qui code pour l'inhibiteur de la C1 estérase (C1EI) humain, facultativement dans une composition, pour utilisation dans le traitement ou la prévention de l'angio-œdème héréditaire chez un mammifère.

2. Le vecteur AAV pour utilisation de la revendication 1, sachant que le vecteur AAV est administré au mammifère une fois avant et/ou après l'apparition de l'œdème.

3. Le vecteur AAV pour utilisation de la revendication 2, sachant que le vecteur AAV est administré au mammifère deux fois ou plus avant et/ou après l'apparition de l'œdème.

4. Le vecteur AAV pour utilisation de l'une quelconque des revendications 1 à 3, sachant que le mammifère est un humain.

5. Le vecteur AAV pour utilisation de l'une quelconque des revendications 1 à 4, sachant que le vecteur AAV est un virus adéno-associé non humain.

6. Le vecteur AAV pour utilisation de la revendication 5, sachant que le virus adéno-associé non humain est un virus adéno-associé du macaque rhésus, facultativement un virus adéno-associé de sérotype rh.10.

7. Le vecteur AAV pour utilisation de l'une quelconque des revendications 1 à 6, sachant que le promoteur est un promoteur spécifique d'un type cellulaire, un promoteur inductible, ou un promoteur constitutivement actif, facultativement un promoteur spécifique d'une cellule, un promoteur inductible, ou un promoteur de l'actine bêta de poulet.

8. Le vecteur AAV pour utilisation de la revendication 7, sachant que le promoteur est un promoteur de l'actine bêta de poulet, et le vecteur AAV comprend en outre une séquence amplificatrice du cytomégalovirus (CMV).

9. Le vecteur AAV pour utilisation de l'une quelconque des revendications 1 à 8, sachant que le vecteur AAV comprend une séquence d'acides nucléiques qui code pour C1EI comprenant la SEQ ID N° 1.

10. Le vecteur AAV pour utilisation de l'une quelconque des revendications 1 à 9, sachant que le vecteur AAV est administré au mammifère à titre prophylactique.

11. Le vecteur AAV pour utilisation de l'une quelconque des revendications 1 à 10, sachant que le vecteur AAV est contenu dans une composition formulée pour une administration intraorale, intramusculaire, transdermique, intraveineuse, intra-artérielle, sous-cutanée, intradermique, ou intrapéritonéale.
